(19) Europäisches Patentamt
European Patent Office
Office européen des brevets

(11) EP 0 711 770 A1

# (12) EUROPÄISCHE PATENTANMELDUNG

(43) Veröffentlichungstag:
**15.05.1996 Patentblatt 1996/20**

(51) Int. Cl.[6]: **C07D 413/06**, C07D 417/06, A61K 31/40

(21) Anmeldenummer: **95116889.7**

(22) Anmeldetag: **26.10.1995**

(84) Benannte Vertragsstaaten:
**AT BE CH DE DK ES FR GB GR IE IT LI LU NL PT SE**

(30) Priorität: **08.11.1994 DE 4439846**

(71) Anmelder: **MERCK PATENT GmbH**
**D-64293 Darmstadt (DE)**

(72) Erfinder:
- **Gante, Joachim, Prof.**
  **D-64291 Darmstadt (DE)**
- **Juraszyk, Horst, Dr.**
  **D-64342 Seeheim (DE)**
- **Raddatz, Peter, Dr.**
  **D-64342 Seeheim (DE)**
- **Wurziger, Hans, Dr.**
  **D-64291 Darmstadt (DE)**
- **Bernotat-Danielowski, Sabine, Dr.**
  **D-61231 Bad Nauheim (DE)**
- **Melzer, Guido, Dr.**
  **D-65719 Hofheim/Ts. (DE)**

## (54) Adhäsionsrezeptor-Antagonisten

(57) Verbindungen der Formel I

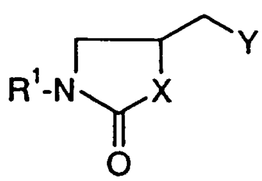

I,

worin $R^1$ und Y die angegebenen Bedeutungen besitzen, sowie deren physiologisch unbedenklichen Salze, hemmen die Bindung von Fibrinogen an den entsprechenden Rezeptor und können zur Behandlung von Thrombosen, Osteoporosen, Tumorerkrankungen, Apoplexie, Herzinfarkt, Entzündungen, Arteriosklerose und osteolytischen Erkrankungen eingesetzt werden.

EP 0 711 770 A1

## Beschreibung

Die Erfindung betrifft Verbindungen der Formel I

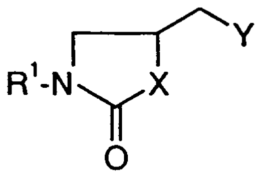

I

worin

X O, S, NH oder NA,

Y einen durch $R^2$ substituierten Aziridino-, Azetidino-, Pyrrolidino-, Piperidino-, Hexahydroazepino- oder Piperazinorest,

$R^1$

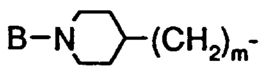

oder

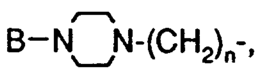

$R^2$ $-C_rH_{2r}-COOR^3$,

$R^3$ H, A oder Ar,

A Alkyl mit 1 bis 6 C-Atomen,

B H, A, Cycloalkyl mit 3 bis 7 C-Atomen, $Ar-C_kH_{2k}-$ oder Amidino,

Ar einen unsubstituierten oder ein- oder zweifach durch A, Cl, Br, I, $NO_2$, CN, OA, OH, $NH_2$, NHA und/oder $NA_2$ substituierten Phenyl- oder Benzyl-Rest,

k 1, 2, 3 oder 4,

m und r jeweils unabhängig voneinander 0, 1, 2, 3 oder 4

und

n 2, 3 oder 4

bedeuten,
sowie ihre physiologisch unbedenklichen Salze.

Ähnliche Verbindungen sind aus der EP-A1-0 381 033 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Diese Aufgabe wurde durch die Erfindung gelöst. Es wurde gefunden, daß die Verbindungen der Formel I sowie ihre Solvate und Salze bei guter Verträglichkeit wertvolle pharmakologische Eigenschaften besitzen. Insbesondere hemmen sie die Bindung von Fibrinogen, Fibronectin und des von-Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Ischämien, Entzündungen, Arteriosklerose und von akutem Nierenversagen verwendet werden. Ferner haben die Verbindungen einen Effekt auf Tumorzellen, indem sie deren Metastasierung hemmen. Somit können sie auch als Anti-Tumor-Mittel eingesetzt werden.

Es gibt Hinweise, daß Tumorzellen durch Mikrothrombi in die Gefäße gelangen und somit vor der Detektion durch die Zellen des Immunsystems geschützt sind. Ebenso wirken Mikrothrombi unterstützend auf die Bindung der Tumorzellen an die Gefäßwände. Da die Bildung der Mikrothrombi im Zusammenhang mit der Fibrinogen-Bindung zum Fibrinogen-Rezeptor (Glycoprotein IIb/IIIa) steht, gelten Fibrinogen-Bindungs-Inhibitoren ebenfalls als Metastase-Inhibitoren.

Die Verbindungen eignen sich zudem als antimikrobielle Wirkstoffe, die Infektionen, wie sie beispielsweise durch Bakterien, Pilze oder Hefen ausgelöst werden, verhindern können. Die Substanzen können daher vorzugsweise als begleitende antimikrobielle Wirkstoffe gegeben werden, wenn Eingriffe an Organismen vorgenommen werden, bei denen körperfremde Stoffe, wie z.B. Biomaterialien, Implantate, Katheter oder Herzschrittmacher, eingesetzt werden. Sie wirken als Antiseptika. Antimikrobielle Aktivitäten der Verbindungen können z.B. nach der Methode von P. Valentin-Weigand et al., beschrieben in Infection and Immunity, 2851-2855 (1988), nachgewiesen werden.

Die anderen Eigenschaften der Verbindungen können nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, 1962) nachweisen.

Gegenstand der Erfindung ist ferner ein Verfahren zur Herstellung einer Verbindung der angegebenen Formel I sowie von deren Salzen, dadurch gekennzeichnet, daß man

(a) eine Verbindung der Formel I aus einem ihrer funktionellen Derivate durch Behandeln mit einem solvolysierenden oder hydrogenolysierenden Mittel in Freiheit setzt, oder daß man

(b) eine Verbindung der Formel II

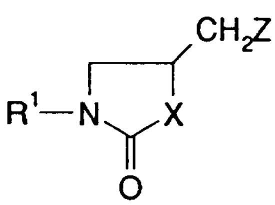

II

worin

Z Cl, Br, I oder eine reaktionsfähig veresterte OH-Gruppe

bedeutet, und

$R^1$ und X die oben angegebenen Bedeutungen haben,

mit einer Verbindung der Formel III

$$H\text{-}Y \qquad III,$$

worin

Y die angegebene Bedeutung besitzt,

umsetzt, oder daß man

(c) eine Verbindung der Formel IV

$$R^1\text{-}NH\text{-}CH_2\text{-}CH(XH)\text{-}CH_2\text{-}Y \qquad IV$$

worin

$R^1$, X und Y die angegebenen Bedeutungen haben,

mit einem reaktionsfähigen Derivat der Kohlensäure umsetzt, oder daß man

(d) eine Verbindung der Formel V

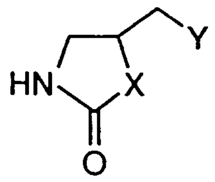

V,

worin

X und Y die angegebenen Bedeutungen haben, mit einer

Verbindung der Formel VI

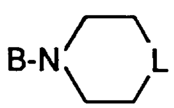

VI,

worin

B die angegebene Bedeutung hat

und

L

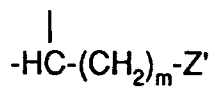

oder >N-$(CH_2)_n$-Z',

wobei

Z' Cl, Br, I, OA, OH oder eine reaktionsfähige veresterte OH-Gruppe bzw. leicht nucleophil substituierbare Abgangsgruppe bedeutet,

umsetzt, oder daß man

(e) (einen) Rest(e) $R^1$ und/oder Y in (einen) andere(n) Rest(e) $R^1$ und/oder Y umwandelt, und/oder daß man

(f) eine Verbindung der Formel I durch Behandeln mit einer Säure oder Base in eines ihrer Salze überführt.

Die Verbindungen der Formel I besitzen mindestens ein chirales Zentrum und können daher in mehreren enantiomeren Formen auftreten. Alle diese Formen (z.B. D- und L-Formen) und deren Gemische (z.B. die DL-Formen) sind in der Formel I eingeschlossen.

Vor- und nachstehend haben die Reste bzw. Parameter A, B, L, X, Y, Z, Z', $R^1$ bis $R^3$, Ar, k, m, n und r die bei den Formeln I bis VI angegebenen Bedeutungen, falls nicht ausdrücklich etwas anderes angegeben ist. Falls mehrere gleich bezeichnete Gruppen im Molekül vorhanden sind, können sie unabhängig voneinander, verschiedene Definitionen annehmen.

In den vorstehenden Formeln hat die Gruppe A 1-6, vorzugsweise 1, 2, 3 oder 4 C-Atome. Im einzelnen bedeutet A vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl.

$R^1$ ist vorzugsweise ein 4-Piperidyl-, 1-Benzyl-4-piperidyl-, 4-Piperidylmethyl-, 4-Piperidylethyl-, 1-Amidino-4-piperidyl-, 1-Amidino-4-piperidylmethyl-, 4-Piperazinylethyl-, 4-Piperazinylpropyl-, 4-Piperazinylbutyl-, 1-Amidino-4-piperazinylethyl- oder ein 1-Amidino-4-piperazinylpropyl-Rest.

$R^2$ steht vorzugsweise für $-(CH_2)_2-COOR^3$, $-(CH_2)_3-COOR^3$ oder $-CH_2-COOR^3$, wobei $R^3$ bevorzugt Wasserstoff, Methyl, Ethyl oder Benzyl bedeutet.

Ar ist vorzugsweise unsubstituiertes Benzyl.

X steht vorzugsweise für Sauerstoff. Y bedeutet besonders bevorzugt Piperazinyl oder Piperidyl.

Die Parameter k und m sind vorzugsweise 0 oder 1. Der Parameter n ist vorzugsweise 2 oder 3, und r bedeutet bevorzugt 1, 2 oder 3.

Unter den Verbindungen der Formel I sind diejenigen bevorzugt, in denen mindestens einer der angegebenen Reste, Gruppen und/oder Parameter eine der angegebenen bevorzugten Bedeutungen hat. Einige Gruppen von bevorzugten Verbindungen sind diejenigen der Formeln Ia bis Ih, die der Formel I entsprechen, worin jedoch

in Ia X Sauerstoff und $R^1$ 4-Piperidyl bedeutet;

in Ib X Sauerstoff und $R^1$ 1-Amidino-4-piperidyl bedeutet;

in Ic X Sauerstoff und $R^1$ 1-Benzyl-4-piperidyl bedeutet;

in Id X Sauerstoff und $R^1$ 4-Piperidylmethyl, -ethyl oder -propyl bedeutet;

in Ie X Sauerstoff und $R^1$ 1-Amidino-4-piperidylmethyl, -ethyl oder -propyl bedeutet;

in If X Sauerstoff und $R^1$ 1-Benzyl-4-piperidylmethyl, -ethyl oder -propyl bedeutet;

in Ig X Sauerstoff und $R^1$ 1-Piperazinylethyl-, 1-Amidino-4-piperazinylethyl oder 1-Benzyl-4-piperazinylethyl bedeutet;

in Ih X Sauerstoff und $R^1$ 1-Piperazinylpropyl-, 1-Amidino-4-piperazinylpropyl oder 1-Benzyl-4-piperazinylpropyl bedeutet.

Weiterhin sind bevorzugt Verbindungen der Formeln Ii sowie Iai bis Ihi, die den Formeln I bzw. Ia bis Ih entsprechen, worin jedoch zusätzlich

Y 3-$R^2$-azetidino, 2-$R^2$-pyrrolidino, 2-$R^2$-piperidino, 3-$R^2$-piperidino, 4-$R^2$-piperidino, 4-$R^2$-piperazino oder 3-$R^2$-piperazino

und

$R^2$ $-COOR^3$, $-CH_2COOR^3$, $-(CH_2)_2COOR^3$ oder $-(CH_2)_3COOR^3$ bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A1-0 381 033, EP-A1-0 462 960) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die an Stelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkyl-gruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, Sulfoxide wie Dimethylsulfoxid (DMSO), ferner auch Alkohole wie Methanol, Ethanol oder Isopropanol sowie Wasser. Ferner kommen Gemische der vorgenannten Lösungsmittel in Frage.

Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B.

Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können bevorzugt auch durch Reaktion einer Verbindung der Formel II mit einer Base der Formel III erhalten werden. Dabei bedient man sich zweckmäßig der an sich bekannten Methoden der N-Alkylierung.

Die Fluchtgruppe Z bedeutet vorzugsweise Cl, Br, I, $C_1$-$C_6$-Alkylsulfonyloxy wie Methan- oder Ethansulfonyloxy oder $C_6$-$C_{10}$-Arylsulfonyloxy wie Benzol-, p-Toluol- oder 1- oder 2-Naphthalinsulfonyloxy.

Die Reaktion gelingt vorzugsweise in Gegenwart einer zusätzlichen Base, z.B. eines Alkali- oder Erdalkalimetallhydroxids oder carbonats wie Natrium-, Kalium- oder Calciumhydroxid, Natrium-, Kalium- oder Calciumcarbonat, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 200, vorzugsweise zwischen 0 und 120°. Falls die Fluchtgruppe Z von I verschieden ist, empfiehlt sich ein Zusatz eines Iodids wie Kaliumiodid.

Die Ausgangsstoffe der Formel II sind in der Regel neu. Sie können z.B. hergestellt werden durch Reaktion eines substituierten Piperidin- oder Piperazinderivates der Formel $R^1$-$NH_2$ mit einer Verbindung der Formel $R^5CH_2$-$CHR^6$-$CH_2OH$ (worin $R^5$ Z, $R^6$ $XR^7$, $R^7$ eine Schutzgtuppe, $R^5$ und $R^6$ zusammen auch O bedeuten) zu einer Verbindung der Formel $R^1$-NH-$CH_2$-$CHR^8$-$CH_2OH$ (worin $R^8$ $XR^7$ oder OH bedeutet), gegebenenfalls Abspaltung der Schutzgruppe $R^7$ zu Verbindungen der Formel $R^1$-NH-$CH_2$-CH(XH)-$CH_2OH$, Reaktion mit einem Derivat der Kohlensäure wie Diethylcarbonat zu 3-$R^1$-5-hydroxymethyl-2-oxazolidinonen und Umwandlung der Hydroxymethylgruppe in eine $CH_2Z$-Gruppe, z.B. mit $SOCl_2$, $SOBr_2$, Methansulfonylchlorid oder p-Toluolsulfonylchlorid. Die Verbindungen der Formel H-Y (III) sind in der Regel bekannt oder in Analogie zu bekannten Verbindungen herstellbar.

Verbindungen der Formel I können ferner erhalten werden durch Reaktion einer Verbindung der Formel IV (oder eines reaktionsfähigen Derivats davon) mit einem reaktiven Derivat der Kohlensäure.

Als Kohlensäurederivate eignen sich insbesondere Dialkylcarbonate wie Diethylcarbonat, ferner auch Chlorameisensäurealkylester wie Ethyl-chlorformiat. Bevorzugt dient das Kohlensäurederivat, das zweckmäßig im Überschuß eingesetzt wird, auch als Lösungs- bzw. Suspensionsmittel. Es kann aber auch eines der angegebenen Lösungsmittel anwesend sein, sofern es bei dieser Umsetzung inert ist. Weiterhin empfiehlt sich der Zusatz einer Base, insbesondere eines Alkalimetallalkoholats wie Kalium-tert.-butylat. Man arbeitet zweckmäßig bei Reaktionstemperaturen zwischen 0 und 150°, vorzugsweise zwischen 70 und 120°.

Die Ausgangsstoffe der Formel IV sind in der Regel neu. Sie sind z.B. erhältlich durch Funktionalisierung der oben genannten Verbindungen der Formel $R^1$-NH-$CH_2$-CH(XH)-$CH_2OH$ zu Verbindungen der Formel $R^1$-NH-$CH_2$-CH(XH)-$CH_2$-E und Reaktion mit Verbindungen der Formel H-Y (III).

Zur Herstellung von Verbindungen der Formel I, worin $R^1$ eine 1-Amidinopiperidinyl- oder -piperazinylgruppe bedeutet, kann man eine entsprechende Piperidin- oder Piperazinverbindung mit einem amidinierenden Mittel behandeln. Als amidinierendes Mittel ist 1-Amidino-3,5-dimethylpyrazol bevorzugt, das insbesondere in Form seines Nitrats eingesetzt wird. Man arbeitet zweckmäßig unter Zusatz einer Base wie Triethylamin oder Ethyl-diisopropylamin in einem inerten Lösungsmittel oder Lösungsmittelgemisch, z.B. Wasser/Dioxan bei Temperaturen zwischen 0 und 120°, vorzugsweise 60 und 120°.

Ferner kann man eine Verbindung der Formel I herstellen, indem man eine Verbindung der Formel V mit einem Piperidin- oder Piperazinderivat der Formel VI umsetzt.

Verbindungen der Formel V können durch Umsetzung einer Verbindung der Formel $H_2N$-$CH_2$-CH(XH)-$CH_2$-Y, worin X und Y die bereits angegebenen Bedeutungen haben, mit einem reaktiven Kohlensäurederivat erhalten werden. Die Reaktionsbedingungen und die geeigneten Kohlensäurederivate entsprechen den zuvor gemachten Angaben.

Die Verbindungen der Formel VI sind in der Regel bekannt oder können nach an sich bekannten Methoden erhalten werden, diejenigen mit $B \neq H$ z.B. durch Funktionalisierung entsprechender Piperidin- oder Piperazinderivate der Formel VI (B=H).

Die Umsetzungen können zweckmäßig nach den an sich bekannten Methoden der N-Alkylierung von Aminen, insbesondere cyclischer Amine, wie sie zuvor für die Verbindungen II und III beschrieben wurden, erhalten werden.

Weiterhin ist es möglich, in einer Verbindung der Formel I einen oder beide der Reste $R^1$ und/oder Y in (einen) andere(n) Rest(e) $R^1$ und/oder Y umzuwandeln.

Insbesondere kann man Carboxylgruppen verestern, Estergruppen spalten, Benzylgruppen hydrogenolytisch entfernen oder Aminogruppen mit einem amidinierenden Mittel behandeln. Ferner kann man konventionelle Amino- oder Hydroxyschutzgruppen einführen oder abspalten.

Zur Veresterung kann man eine Säure der Formel I ($R^3$ = H) mit einem Überschuß eines Alkohols der Formel $R^3$-OH ($R^3$ = A oder Benzyl) behandeln, zweckmäßig in Gegenwart einer starken Säure wie Salzsäure oder Schwefelsäure bei Temperaturen zwischen 0 und 100, vorzugsweise 20 und 50°.

Umgekehrt kann ein Ester der Formel I ($R^3$ = A oder Benzyl), in die entsprechende Säure der Formel I ($R^3$ = H) umgewandelt werden, zweckmäßig durch Solvolyse nach einer der oben angegebenen Methoden, z.B. mit NaOH oder KOH in Wasser-Dioxan bei Temperaturen zwischen 0 und 40°, vorzugsweise 10 und 30°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Es ist auch möglich, Carbonsäuren der Formel I ( $R^3$ = H ) durch Umsetzung mit entsprechenden Basen in ihre Metall- oder Ammoniumsalze umzuwandeln, z.B. ihre Natrium-, Kalium- oder Calciumsalze.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch-aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoyl-phenyl-glycin) gefüllten Säule; als Laufmittel eignet sich z.B. ein Gemisch Hexan/Isopropanol/ Acetonitril, z.B. im Volumenverhältnis 82:15:3.

Natürlich ist es auch möglich, optisch-aktive Verbindungen der Formel I nach den oben beschriebenen Methoden zu erhalten, indem man Ausgangsstoffe (z.B. solche der Formel II) verwendet, die bereits optisch-aktiv sind.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Übetrügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Pharmaka, insbesondere aber in Analogie zu den in der EP-A-459 256 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein; filtriert über einen Ionenaustauscher, trennt ab, trocknet über Natriumsulfat, lyophilisiert gegebenenfalls, dampft ein und reinigt durch Chromatographie an Kieselgel und/oder Kristallisation.

**Beispiel 1**

Zu einer Lösung von 1,6 g 3-Piperazino-propansäuremethylester ("A") [erhältlich durch Umsetzung von 3-Chlorpropansäuremethylester mit Piperazin] in 20 ml DMF gibt man 3,0 g 3-(1-BOC-4-Piperidyl)-5-methansulfonyloxy-methyl-oxazolidin-2-on [erhältlich durch Reaktion von 1-BOC-4-Amino-piperidin mit 2,3-Epoxypropan-1-ol zu 4-(2,3-Dihydroxy-propyl-amino)-piperidin, Umsetzung mit Diethylcarbonat in Gegenwart von K-tert.-butylat zu 3-(1-BOC-4-Piperidyl)-5-hydroxymethyl-oxazolidin-2-on und anschließende Veresterung mit Methansulfonylchlorid], gelöst in 10 ml DMF, hinzu und rührt 60 Min. bei Raumtemperatur. Nach Entfernung des Lösungsmittels und üblicher Aufarbeitung erhält man das 3-(1-BOC-4-Piperidyl)-5-[4-(2-methoxycarbonyl-ethyl)-piperazino]-methyl-oxazolidin-2-on.

Analog erhält man durch Umsetzung von "A"

mit 3-(1-Benzyl-4-piperidyl)-5-methansulfonyloxy-methyl-oxazolidin-2-on das
3-(1-Benzyl-4-piperidyl)-5-[4-(2-methoxycarbonyl-ethyl)-piperazino]-methyl-oxazolidin-2-on, F. 86-87°;
mit 3-(1-N-BOC-Amidino-4-piperidyl)-5-methansulfonyloxy-methyl-oxazolidin-2-on das
3-(1-N-BOC-Amidino-4-piperidyl)-5-[4-(2-methoxycarbonyl-ethyl)-piperazino]-methyl-oxazolidin-2-on;
mit 3-(1-Benzyl-4-piperidylmethyl)-5-methansulfonyloxy-methyl-oxazolidin-2-on das
3-(1-Benzyl-4-piperidylmethyl)-5-[4-(2-methoxycarbonyl-ethyl)-piperazino]-methyl-oxazolidin-2-on;
mit 3-(1-N-BOC-Amidino-4-piperidylmethyl)-5-methansulfonyloxy-methyl-oxazolidin-2-on das
3-(1-N-BOC-Amidino-4-piperidylmethyl)-5-[4-(2-methoxycarbonyl-ethyl)-piperazino]-methyl-oxazolidin-2-on;
mit 3-[2-(1-Benzyl-4-piperidyl)-ethyl]-5-methansulfonyloxy-methyl-oxazolidin-2-on das
3-[2-(1-Benzyl-4-piperidyl)-ethyl]-5-[4-(2-methoxycarbonyl-ethyl)-piperazino]-methyl-oxazolidin-2-on;
mit 3-[2-(1-N-BOC-Amidino-4-piperidyl)-ethyl]-5-methansulfonyloxy-methyl-oxazolidin-2-on das
3-[2-(1-N-BOC-Amidino-4-piperidyl)-ethyl]-5-[4-(2-methoxycarbonyl-ethyl)-piperazino]-methyl-oxazolidin-2-on;
mit 3-[3-(1-Benzyl-4-piperidyl)-propyl]-5-methansulfonyloxy-methyl-oxazolidin-2-on das
3-[3-(1-Benzyl-4-piperidyl)-propyl]-5-[4-(2-methoxycarbonyl-ethyl)-piperazino]-methyl-oxazolidin-2-on;
mit 3-[3-(1-N-BOC-Amidino-4-piperidyl)-propyl]-5-methansulfonyloxy-methyl-oxazolidin-2-on das
3-[3-(1-N-BOC-Amidino-4-piperidyl)-propyl]-5-[4-(2-methoxycarbonyl-ethyl)-piperazino]-methyl-oxazolidin-2-on;
mit 3-[4-(1-Benzyl-4-piperidyl)-butyl]-5-methansulfonyloxy-methyl-oxazolidin-2-on das
3-[4-(1-Benzyl-4-piperidyl)-butyl]-5-[4-(2-methoxycarbonyl-ethyl)-piperazino]-methyl-oxazolidin-2-on;
mit 3-[4-(1-N-BOC-Amidino-4-piperidyl)-butyl]-5-methansulfonyloxy-methyl-oxazolidin-2-on das
3-[4-(1-N-BOC-Amidino-4-piperidyl)-butyl]-5-[4-(2-methoxycarbonyl-ethyl)-piperazino]-methyl-oxazolidin-2-on;
mit 3-[2-(1-Benzyl-4-piperazinyl)-ethyl]-5-methansulfonyloxy-methyl-oxazolidin-2-on das
3-[2-(1-Benzyl-4-piperazinyl)-ethyl]-5-[4-(2-methoxycarbonyl-ethyl)-piperazino]-methyl-oxazolidin-2-on;
mit 3-[2-(1-N-BOC-Amidino-4-piperazinyl)-ethyl]-5-methansulfonyloxy-methyl-oxazolidin-2-on das
3-[2-(1-N-BOC-Amidino-4-piperazinyl)-ethyl]-5-[4-(2-methoxycarbonyl-ethyl)-piperazino]-methyl-oxazolidin-2-on;
mit 3-[3-(1-Benzyl-4-piperazinyl)-propyl]-5-methansulfonyloxy-methyl-oxazolidin-2-on das
3-[3-(1-Benzyl-4-piperazinyl)-propyl]-5-[4-(2-methoxycarbonyl-ethyl)-piperazino]-methyl-oxazolidin-2-on;
mit 3-[3-(1-N-BOC-Amidino-4-piperazinyl)-propyl]-5-methansulfonyloxy-methyl-oxazolidin-2-on das
3-[3-(1-N-BOC-Amidino-4-piperazinyl)-propyl]-5-[4-(2-methoxycarbonyl-ethyl)-piperazino]-methyl-oxazolidin-2-on;
mit 3-[4-(1-Benzyl-4-piperazinyl)-butyl]-5-methansulfonyloxy-methyl-oxazolidin-2-on das
3-[4-(1-Benzyl-4-piperazinyl)-butyl]-5-[4-(2-methoxycarbonyl-ethyl)-piperazino]-methyl-oxazolidin-2-on;
mit 3-[4-(1-N-BOC-Amidino-4-piperazinyl)-butyl]-5-methansulfonyloxy-methyl-oxazolidin-2-on das
3-[4-(1-N-BOC-Amidino-4-piperazinyl)-butyl]-5-[4-(2-methoxycarbonyl-ethyl)-piperazino]-methyl-oxazolidin-2-on;
mit 3-(1-Methyl-4-piperidyl)-5-methansulfonyloxy-methyl-oxazolidin-2-on das
3-(1-Methyl-4-piperidyl)-5-[4-(2-methoxycarbonyl-ethyl)-piperazino]-methyl-oxazolidin-2-on;
mit 3-(1-Isopropyl-4-piperidyl)-5-methansulfonyloxy-methyl-oxazolidin-2-on das
3-(1-Isopropyl-4-piperidyl)-5-[4-(2-methoxycarbonyl-ethyl)-piperazino]-methyl-oxazolidin-2-on;
mit 3-(1-tert.-Butyl-4-piperidylmethyl)-5-methansulfonyloxy-methyl-oxazolidin-2-on das
3-(1-tert.-Butyl-4-piperidylmethyl)-5-[4-(2-methoxycarbonyl-ethyl)-piperazino]-methyl-oxazolidin-2-on;
mit 3-(1-Ethyl-4-piperidylmethyl)-5-methansulfonyloxy-methyl-oxazolidin-2-on das
3-(1-Ethyl-4-piperidylmethyl)-5-[4-(2-methoxycarbonyl-ethyl)-piperazino]-methyl-oxazolidin-2-on;
mit 3-[2-(1-Isopropyl-4-piperidyl)-ethyl]-5-methansulfonyloxy-methyl-oxazolidin-2-on das
3-[2-(1-Isopropyl-4-piperidyl)-ethyl]-5-[4-(2-methoxycarbonyl-ethyl)-piperazino]-methyl-oxazolidin-2-on;
mit 3-[2-(1-Cyclohexyl-4-piperidyl)-ethyl]-5-methansulfonyloxy-methyl-oxazolidin-2-on das

3-[2-(1-Cyclohexyl-4-piperidyl)-ethyl]-5-[4-(2-methoxycarbonyl-ethyl)-piperazino]-methyl-oxazolidin-2-on;
mit 3-[3-(1-Methyl-4-piperidyl)-propyl]-5-methansulfonyloxy-methyl-oxazolidin-2-on das
3-[3-(1-Methyl-4-piperidyl)-propyl]-5-[4-(2-methoxycarbonyl-ethyl)-piperazino]-methyl-oxazolidin-2-on;
mit 3-[3-(1-Cyclopentyl-4-piperidyl)-propyl]-5-methansulfonyloxy-methyl-oxazolidin-2-on das
3-[3-(1-Cyclopentyl-4-piperidyl)-propyl]-5-[4-(2-methoxycarbonyl-ethyl)-piperazino]-methyl-oxazolidin-2-on;
mit 3-[4-(1-tert.-Butyl-4-piperidyl)-butyl]-5-methansulfonyloxy-methyl-oxazolidin-2-on das
3-[4-(1-tert.-Butyl-4-piperidyl)-butyl]-5-[4-(2-methoxycarbonyl-ethyl)-piperazino]-methyl-oxazolidin-2-on;
mit 3-[4-(1-Cyclopropyl-4-piperidyl)-butyl]-5-methansulfonyloxy-methyl-oxazolidin-2-on das
3-[4-(1-Cyclopropyl-4-piperidyl)-butyl]-5-[4-(2-methoxycarbonyl-ethyl)-piperazino]-methyl-oxazolidin-2-on;
mit 3-[2-(1-Methyl-4-piperazinyl)-ethyl]-5-methansulfonyloxy-methyl-oxazolidin-2-on das
3-[2-(1-Methyl-4-piperazinyl)-ethyl]-5-[4-(2-methoxycarbonyl-ethyl)-piperazino]-methyl-oxazolidin-2-on;
mit 3-[2-(1-Cyclopropyl-4-piperazinyl)-ethyl]-5-methansulfonyloxy-methyl-oxazolidin-2-on das
3-[2-(1-Cyclopropyl-4-piperazinyl)-ethyl]-5-[4-(2-methoxycarbonyl-ethyl)-piperazino]-methyl-oxazolidin-2-on;
mit 3-[3-(1-Ethyl-4-piperazinyl)-propyl]-5-methansulfonyloxy-methyl-oxazolidin-2-on das
3-[3-(1-Ethyl-4-piperazinyl)-propyl]-5-[4-(2-methoxycarbonyl-ethyl)-piperazino]-methyl-oxazolidin-2-on;
mit 3-[3-(1-Isopropyl-4-piperazinyl)-propyl]-5-methansulfonyloxy-methyl-oxazolidin-2-on das
3-[3-(1-Isopropyl-4-piperazinyl)-propyl]-5-[4-(2-methoxycarbonyl-ethyl)-piperazino]-methyl-oxazolidin-2-on;
mit 3-[4-(1-Propyl-4-piperazinyl)-butyl]-5-methansulfonyloxy-methyl-oxazolidin-2-on das
3-[4-(1-Propyl-4-piperazinyl)-butyl]-5-[4-(2-methoxycarbonyl-ethyl)-piperazino]-methyl-oxazolidin-2-on;
mit 3-[4-(1-Cyclohexyl-4-piperazinyl)-butyl]-5-methansulfonyloxy-methyl-oxazolidin-2-on das
3-[4-(1-Cyclohexyl-4-piperazinyl)-butyl]-5-[4-(2-methoxycarbonyl-ethyl)-piperazino]-methyl-oxazolidin-2-on.

## Beispiel 2

Analog Beispiel 1 erhält man durch Umsetzung von 2,2 g 3-Piperazinopropansäurebenzylester ("B") mit 2,6 g 3-(1-Benzyl-4-piperidyl)-5-methansulfonyloxy-methyl-oxazolidin-2-on das 3-(1-Benzyl-4-piperidyl)-5-[4-(2-benzyloxycarbonyl-ethyl)-piperazino]-methyl-oxazolidin-2-on, F. 91-92°.

Analog erhält man durch Umsetzung von "B"
mit 3-(1-Benzyl-4-piperidylmethyl)-5-methansulfonyloxy-methyl-oxazolidin-2-on das
3-(1-Benzyl-4-piperidylmethyl)-5-[4-(2-benzyloxycarbonyl-ethyl)-piperazino]-methyl-oxazolidin-2-on;
mit 3-[2-(1-Benzyl-4-piperidyl)-ethyl]-5-methansulfonyloxy-methyl-oxazolidin-2-on das
3-[2-(1-Benzyl-4-piperidyl)-ethyl]-5-[4-(2-benzyloxycarbonyl-ethyl)-piperazino]-methyl-oxazolidin-2-on;
mit 3-[3-(1-Benzyl-4-piperidyl)-propyl]-5-methansulfonyloxy-methyl-oxazolidin-2-on das
3-[3-(1-Benzyl-4-piperidyl)-propyl]-5-[4-(2-benzyloxycarbonyl-ethyl)-piperazino]-methyl-oxazolidin-2-on;
mit 3-[4-(1-Benzyl-4-piperidyl)-butyl]-5-methansulfonyloxy-methyl-oxazolidin-2-on das
3-[4-(1-Benzyl-4-piperidyl)-butyl]-5-[4-(2-benzyloxycarbonyl-ethyl)-piperazino]-methyl-oxazolidin-2-on.

## Beispiel 3

Analog Beispiel 1 erhält man ausgehend von 2-Piperazinoessigsäuremethylester [erhältlich durch Umsetzung von α-Chloressigsäuremethylester mit Piperazin] durch Umsetzung mit 3-(1-BOC-4-piperidyl)-5-methansulfonyloxy-methyl-oxazolidin-2-on nach üblicher Aufarbeitung das 3-(1-BOC-4-piperidyl)-5-[4-(methoxycarbonyl-methyl)-piperazino]-methyl-oxazolidin-2-on.

Analog erhält man durch Umsetzung von 2-Piperazinoessigsäuremethylester
mit 3-(1-Benzyl-4-piperidyl)-5-methansulfonyloxy-methyl-oxazolidin-2-on das
3-(1-Benzyl-4-piperidyl)-5-[4-(methoxycarbonyl-methyl)-piperazino]-methyl-oxazolidin-2-on;
mit 3-(1-N-BOC-Amidino-4-piperidyl)-5-methansulfonyloxy-methyl-oxazolidin-2-on das
3-(1-N-BOC-Amidino-4-piperidyl)-5-[4-(methoxycarbonyl-methyl)-piperazino]-methyl-oxazolidin-2-on;
mit 3-(1-Benzyl-4-piperidylmethyl)-5-methansulfonyloxy-methyl-oxazolidin-2-on das
3-(1-Benzyl-4-piperidylmethyl)-5-[4-(methoxycarbonyl-methyl)-piperazino]-methyl-oxazolidin-2-on;
mit 3-(1-N-BOC-Amidino-4-piperidylmethyl)-5-methansulfonyloxy-methyl-oxazolidin-2-on das
3-(1-N-BOC-Amidino-4-piperidylmethyl)-5-[4-(methoxycarbonyl-methyl)-piperazino]-methyl-oxazolidin-2-on;
mit 3-[2-(1-Benzyl-4-piperidyl)-ethyl]-5-methansulfonyloxy-methyl-oxazolidin-2-on das
3-[2-(1-Benzyl-4-piperidyl)-ethyl]-5-[4-(methoxycarbonyl-methyl)-piperazino]-methyl-oxazolidin-2-on;
mit 3-[2-(1-N-BOC-Amidino-4-piperidyl)-ethyl]-5-methansulfonyloxy-methyl-oxazolidin-2-on das
3-[2-(1-N-BOC-Amidino-4-piperidyl)-ethyl]-5-[4-(methoxycarbonyl-methyl)-piperazino]-methyl-oxazolidin-2-on;
mit 3-[3-(1-Benzyl-4-piperidyl)-propyl]-5-methansulfonyloxy-methyl-oxazolidin-2-on das
3-[3-(1-Benzyl-4-piperidyl)-propyl]-5-[4-(methoxycarbonyl-methyl)-piperazino]-methyl-oxazolidin-2-on;
mit 3-[3-(1-N-BOC-Amidino-4-piperidyl)-propyl]-5-methansulfonyloxy-methyl-oxazolidin-2-on das

3-[3-(1-N-BOC-Amidino-4-piperidyl)-propyl]-5-[4-(methoxycarbonyl-methyl)-piperazino]-methyl-oxazolidin-2-on;
mit 3-[4-(1-Benzyl-4-piperidyl)-butyl]-5-methansulfonyloxy-methyl-oxazolidin-2-on das
3-[4-(1-Benzyl-4-piperidyl)-butyl]-5-[4-(methoxycarbonyl-methyl)-piperazino]-methyl-oxazolidin-2-on;
mit 3-[4-(1-N-BOC-Amidino-4-piperidyl)-butyl]-5-methansulfonyloxy-methyl-oxazolidin-2-on das
3-[4-(1-N-BOC-Amidino-4-piperidyl)-butyl]-5-[4-(methoxycarbonyl-methyl)-piperazino]-methyl-oxazolidin-2-on;
mit 3-[2-(1-Benzyl-4-piperazinyl)-ethyl]-5-methansulfonyloxy-methyl-oxazolidin-2-on das
3-[2-(1-Benzyl-4-piperazinyl)-ethyl]-5-[4-(methoxycarbonyl-methyl)-piperazino]-methyl-oxazolidin-2-on;
mit 3-[2-(1-N-BOC-Amidino-4-piperazinyl)-ethyl]-5-methansulfonyloxy-methyl-oxazolidin-2-on das
3-[2-(1-N-BOC-Amidino-4-piperazinyl)-ethyl]-5-[4-(methoxycarbonyl-methyl)-piperazino]-methyl-oxazolidin-2-on;
mit 3-[3-(1-Benzyl-4-piperazinyl)-propyl]-5-methansulfonyloxy-methyl-oxazolidin-2-on das
3-[3-(1-Benzyl-4-piperazinyl)-propyl]-5-[4-(methoxycarbonyl-methyl)-piperazino]-methyl-oxazolidin-2-on;
mit 3-[3-(1-N-BOC-Amidino-4-piperazinyl)-propyl]-5-methansulfonyloxy-methyl-oxazolidin-2-on das
3-[3-(1-N-BOC-Amidino-4-piperazinyl)-propyl]-5-[4-(methoxycarbonyl-methyl)-piperazino]-methyl-oxazolidin-2-on;
mit 3-[4-(1-Benzyl-4-piperazinyl)-butyl]-5-methansulfonyloxy-methyl-oxazolidin-2-on das
3-[4-(1-Benzyl-4-piperazinyl)-butyl]-5-[4-(methoxycarbonyl-methyl)-piperazino]-methyl-oxazolidin-2-on;
mit 3-[4-(1-N-BOC-Amidino-4-piperazinyl)-butyl]-5-methansulfonyloxy-methyl-oxazolidin-2-on das
3-[4-(1-N-BOC-Amidino-4-piperazinyl)-butyl]-5-[4-(methoxycarbonyl-methyl)-piperazino]-methyl-oxazolidin-2-on;
mit 3-(1-Methyl-4-piperidyl)-5-methansulfonyloxy-methyl-oxazolidin-2-on das
3-(1-Methyl-4-piperidyl)-5-[4-(methoxycarbonyl-methyl)-piperazino]-methyl-oxazolidin-2-on;
mit 3-(1-Isopropyl-4-piperidyl)-5-methansulfonyloxy-methyl-oxazolidin-2-on das
3-(1-Isopropyl-4-piperidyl)-5-[4-(methoxycarbonyl-methyl)-piperazino]-methyl-oxazolidin-2-on;
mit 3-(1-tert.-Butyl-4-piperidylmethyl)-5-methansulfonyloxy-methyl-oxazolidin-2-on das
3-(1-tert.-Butyl-4-piperidylmethyl)-5-[4-(methoxycarbonyl-methyl)-piperazino]-methyl-oxazolidin-2-on;
mit 3-(1-Ethyl-4-piperidylmethyl)-5-methansulfonyloxy-methyl-oxazolidin-2-on das
3-(1-Ethyl-4-piperidylmethyl)-5-[4-(methoxycarbonyl-methyl)-piperazino]-methyl-oxazolidin-2-on;
mit 3-[2-(1-Isopropyl-4-piperidyl)-ethyl]-5-methansulfonyloxy-methyl-oxazolidin-2-on das
3-[2-(1-Isopropyl-4-piperidyl)-ethyl]-5-[4-(methoxycarbonyl-methyl)-piperazino]-methyl-oxazolidin-2-on;
mit 3-[2-(1-Cyclohexyl-4-piperidyl)-ethyl]-5-methansulfonyloxy-methyl-oxazolidin-2-on das
3-[2-(1-Cyclohexyl-4-piperidyl)-ethyl]-5-[4-(methoxycarbonyl-methyl)-piperazino]-methyl-oxazolidin-2-on;
mit 3-[3-(1-Methyl-4-piperidyl)-propyl]-5-methansulfonyloxy-methyl-oxazolidin-2-on das
3-[3-(1-Methyl-4-piperidyl)-propyl]-5-[4-(methoxycarbonyl-methyl)-piperazino]-methyl-oxazolidin-2-on;
mit 3-[3-(1-Cyclopentyl-4-piperidyl)-propyl]-5-methansulfonyloxy-methyl-oxazolidin-2-on das
3-[3-(1-Cyclopentyl-4-piperidyl)-propyl]-5-[4-(methoxycarbonyl-methyl)-piperazino]-methyl-oxazolidin-2-on;
mit 3-[4-(1-tert.-Butyl-4-piperidyl)-butyl]-5-methansulfonyloxy-methyl-oxazolidin-2-on das
3-[4-(1-tert.-Butyl-4-piperidyl)-butyl]-5-[4-(methoxycarbonyl-methyl)-piperazino]-methyl-oxazolidin-2-on;
mit 3-[4-(1-Cyclopropyl-4-piperidyl)-butyl]-5-methansulfonyloxy-methyl-oxazolidin-2-on das
3-[4-(1-Cyclopropyl-4-piperldyl)-butyl]-5-[4-(methoxycarbonyl-methyl)-piperazino]-methyl-oxazolidin-2-on;
mit 3-[2-(1-Methyl-4-piperazinyl)-ethyl]-5-methansulfonyloxy-methyl-oxazolidin-2-on das
3-[2-(1-Methyl-4-piperazinyl)-ethyl]-5-[4-(methoxycarbonyl-methyl)-piperazino]-methyl-oxazolidin-2-on;
mit 3-[2-(1-Cyclopropyl-4-piperazinyl)-ethyl]-5-methansulfonyloxy-methyl-oxazolidin-2-on das
3-[2-(1-Cyclopropyl-4-piperazinyl)-ethyl]-5-[4-(methoxycarbonyl-methyl)-piperazino]-methyl-oxazolidin-2-on;
mit 3-[3-(1-Ethyl-4-piperazinyl)-propyl]-5-methansulfonyloxy-methyl-oxazolidin-2-on das
3-[3-(1-Ethyl-4-piperazinyl)-propyl]-5-[4-(methoxycarbonyl-methyl)-piperazino]-methyl-oxazolidin-2-on;
mit 3-[3-(1-Isopropyl-4-piperazinyl)-propyl]-5-methansulfonyloxy-methyl-oxazolidin-2-on das
3-[3-(1-Isopropyl-4-piperazinyl)-propyl])-5-[4-(methoxycarbonyl-methyl)-piperazino]-methyl-oxazolidin-2-on;
mit 3-[4-(1-Propyl-4-piperazinyl)-butyl]-5-methansulfonyloxy-methyl-oxazolidin-2-on das
3-[4-(1-Propyl-4-piperazinyl)-butyl]-5-[4-(methoxycarbonyl-methyl)-piperazino]-methyl-oxazolidin-2-on;
mit 3-[4-(1-Cyclohexyl-4-piperazinyl)-butyl]-5-methansulfonyloxy-methyl-oxazolidin-2-on das
3-[4-(1-Cyclohexyl-4-piperazinyl)-butyl]-5-[4-(methoxycarbonyl-methyl)-piperazino]-methyl-oxazolidin-2-on.

Analog erhält man durch Umsetzung von 4-Piperazinobutansäuremethylester
mit 3-(1-Benzyl-4-piperidyl)-5-methansulfonyloxy-methyl-oxazolidin-2-on das
3-(1-Benzyl-4-piperidyl)-5-[4-(3-methoxycarbonyl-propyl)-piperazino]-methyl-oxazolidin-2-on;
mit 3-(1-N-BOC-Amidino-4-piperidyl)-5-methansulfonyloxy-methyl-oxazolidin-2-on das
3-(1-N-BOC-Amidino-4-piperidyl)-5-[4-(3-methoxycarbonyl-propyl)-piperazino]-methyl-oxazolidin-2-on;
mit 3-(1-Benzyl-4-piperidylmethyl)-5-methansulfonyloxy-methyl-oxazolidin-2-on das
3-(1-Benzyl-4-piperidylmethyl)-5-[4-(3-methoxycarbonyl-propyl)-piperazino]-methyl-oxazolidin-2-on;

mit 3-(1-N-BOC-Amidino-4-piperidylmethyl)-5-methansulfonyloxy-methyl-oxazolidin-2-on das
3-(1-N-BOC-Amidino-4-piperidylmethyl)-5-[4-(3-methoxycarbonyl-propyl)-piperazino]-methyl-oxazolidin-2-on;
mit 3-[2-(1-Benzyl-4-piperidyl)-ethyl]-5-methansulfonyloxy-methyl-oxazolidin-2-on das
3-[2-(1-Benzyl-4-piperidyl)-ethyl]-5-[4-(3-methoxycarbonyl-propyl)-piperazino]-methyl-oxazolidin-2-on;
mit 3-[2-(1-N-BOC-Amidino-4-piperidyl)-ethyl]-5-methansulfonyloxy-methyl-oxazolidin-2-on das
3-[2-(1-N-BOC-Amidino-4-piperidyl)-ethyl]-5-[4-(3-methoxycarbonyl-propyl)-piperazino]-methyl-oxazolidin-2-on;
mit 3-[3-(1-Benzyl-4-piperidyl)-propyl]-5-methansulfonyloxy-methyl-oxazolidin-2-on das
3-[3-(1-Benzyl-4-piperidyl)-propyl]-5-[4-(3-methoxycarbonyl-propyl)-piperazino]-methyl-oxazolidin-2-on;
mit 3-[3-(1-N-BOC-Amidino-4-piperidyl)-propyl]-5-methansulfonyloxy-methyl-oxazolidin-2-on das
3-[3-(1-N-BOC-Amidino-4-piperidyl)-propyl]-5-[4-(3-methoxycarbonylpropyl)-piperazino]-methyl-oxazolidin-2-on;
mit 3-[4-(1-Benzyl-4-piperidyl)-butyl]-5-methansulfonyloxy-methyl-oxazolidin-2-on das
3-[4-(1-Benzyl-4-piperidyl)-butyl]-5-[4-(3-methoxycarbonyl-propyl)-piperazino]-methyl-oxazolidin-2-on;
mit 3-[4-(1-N-BOC-Amidino-4-piperidyl)-butyl]-5-methansulfonyloxy-methyl-oxazolidin-2-on das
3-[4-(1-N-BOC-Amidino-4-piperidyl)-butyl]-5-[4-(3-methoxycarbonyl-propyl)-piperazino]-methyl-oxazolidin-2-on;
mit 3-[2-(1-Benzyl-4-piperazinyl)-ethyl]-5-methansulfonyloxy-methyl-oxazolidin-2-on das
3-[2-(1-Benzyl-4-piperazinyl)-ethyl]-5-[4-(3-methoxycarbonyl-propyl)-piperazino]-methyl-oxazolidin-2-on;
mit 3-[2-(1-N-BOC-Amidino-4-piperazinyl)-ethyl]-5-methansulfonyloxy-methyl-oxazolidin-2-on das
3-[2-(1-N-BOC-Amidino-4-piperazinyl)-ethyl]-5-[4-(3-methoxycarbonyl-propyl)-piperazino]-methyl-oxazolidin-2-on;
mit 3-[3-(1-Benzyl-4-piperazinyl)-propyl]-5-methansulfonyloxy-methyl-oxazolidin-2-on das
3-[3-(1-Benzyl-4-piperazinyl)-propyl]-5-[4-(3-methoxycarbonyl-propyl)-piperazino]-methyl-oxazolidin-2-on;
mit 3-[3-(1-N-BOC-Amidino-4-piperazinyl)-propyl]-5-methansulfonyloxy-methyl-oxazolidin-2-on das
3-[3-(1-N-BOC-Amidino-4-piperazinyl)-propyl]-5-[4-(3-methoxycarbonyl-propyl)-piperazino]-methyl-oxazolidin-2-on;
mit 3-[4-(1-Benzyl-4-piperazinyl)-butyl]-5-methansulfonyloxy-methyl-oxazolidin-2-on das
3-[4-(1-Benzyl-4-piperazinyl)-butyl]-5-[4-(3-methoxycarbonyl-propyl)-piperazino]-methyl-oxazolidin-2-on;
mit 3-[4-(1-N-BOC-Amidino-4-piperazinyl)-butyl]-5-methansulfonyloxy-methyl-oxazolidin-2-on das
3-[4-(1-N-BOC-Amidino-4-piperazinyl)-butyl]-5-[4-(3-methoxycarbonyl-propyl)-piperazino]-methyl-oxazolidin-2-on;
mit 3-(1-Methyl-4-piperidyl)-5-methansulfonyloxy-methyl-oxazolidin-2-on das
3-(1-Methyl-4-piperidyl)-5-[4-(3-methoxycarbonyl-propyl)-piperazino]-methyl-oxazolidin-2-on;
mit 3-(1-Isopropyl-4-piperidyl)-5-methansulfonyloxy-methyl-oxazolidin-2-on das
3-(1-Isopropyl-4-piperidyl)-5-[4-(3-methoxycarbonyl-propyl)-piperazino]-methyl-oxazolidin-2-on;
mit 3-(1-tert.-Butyl-4-piperidylmethyl)-5-methansulfonyloxy-methyl-oxazolidin-2-on das
3-(1-tert.-Butyl-4-piperidylmethyl)-5-[4-(3-methoxycarbonyl-propyl)-piperazino]-methyl-oxazolidin-2-on;
mit 3-(1-Ethyl-4-piperidylmethyl)-5-methansulfonyloxy-methyl-oxazolidin-2-on das
3-(1-Ethyl-4-piperidylmethyl)-5-[4-(3-methoxycarbonyl-propyl)-piperazino]-methyl-oxazolidin-2-on;
mit 3-[2-(1-Isopropyl-4-piperidyl)-ethyl]-5-methansulfonyloxy-methyl-oxazolidin-2-on das
3-[2-(1-Isopropyl-4-piperidyl)-ethyl]-5-[4-(3-methoxycarbonyl-propyl)-piperazino]-methyl-oxazolidin-2-on;
mit 3-[2-(1-Cyclohexyl-4-piperidyl)-ethyl]-5-methansulfonyloxy-methyl-oxazolidin-2-on das
3-[2-(1-Cyclohexyl-4-piperidyl)-ethyl]-5-[4-(3-methoxycarbonyl-propyl)-piperazino]-methyl-oxazolidin-2-on;
mit 3-[3-(1-Methyl-4-piperidyl)-propyl]-5-methansulfonyloxy-methyl-oxazolidin-2-on das
3-[3-(1-Methyl-4-piperidyl)-propyl]-5-[4-(3-methoxycarbonyl-propyl)-piperazino]-methyl-oxazolidin-2-on;
mit 3-[3-(1-Cyclopentyl-4-piperidyl)-propyl]-5-methansulfonyloxy-methyl-oxazolidin-2-on das
3-[3-(1-Cyclopentyl-4-piperidyl)-propyl]-5-[4-(3-methoxycarbonyl-propyl)-piperazino]-methyl-oxazolidin-2-on;
mit 3-[4-(1-tert.-Butyl-4-piperidyl)-butyl]-5-methansulfonyloxy-methyl-oxazolidin-2-on das
3-[4-(1-tert.-Butyl-4-piperidyl)-butyl]-5-[4-(3-methoxycarbonyl-propyl)-piperazino]-methyl-oxazolidin-2-on;
mit 3-[4-(1-Cyclopropyl-4-piperidyl)-butyl]-5-methansulfonyloxy-methyl-oxazolidin-2-on das
3-[4-(1-Cyclopropyl-4-piperidyl)-butyl]-5-[4-(3-methoxycarbonyl-propyl)-piperazino]-methyl-oxazolidin-2-on;
mit 3-[2-(1-Methyl-4-piperazinyl)-ethyl]-5-methansulfonyloxy-methyl-oxazolidin-2-on das
3-[2-(1-Methyl-4-piperazinyl)-ethyl]-5-[4-(3-methoxycarbonyl-propyl)-piperazino]-methyl-oxazolidin-2-on;
mit 3-[2-(1-Cyclopropyl-4-piperazinyl)-ethyl]-5-methansulfonyloxy-methyl-oxazolidin-2-on das
3-[2-(1-Cyclopropyl-4-piperazinyl)-ethyl]-5-[4-(3-methoxycarbonyl-propyl)-piperazino]-methyl-oxazolidin-2-on;
mit 3-[3-(1-Ethyl-4-piperazinyl)-propyl]-5-methansulfonyloxy-methyl-oxazolidin-2-on das
3-[3-(1-Ethyl-4-piperazinyl)-propyl]-5-[4-(3-methoxycarbonyl-propyl)-piperazino]-methyl-oxazolidin-2-on;
mit 3-[3-(1-Isopropyl-4-piperazinyl)-propyl]-5-methansulfonyloxy-methyl-oxazolidin-2-on das
3-[3-(1-Isopropyl-4-piperazinyl)-propyl]-5-[4-(3-methoxycarbonyl-propyl)-piperazino]-methyl-oxazolidin-2-on;
mit 3-[4-(1-Propyl-4-piperazinyl)-butyl]-5-methansulfonyloxy-methyl-oxazolidin-2-on das

3-[4-(1-Propyl-4-piperazinyl)-butyl]-5-[4-(3-methoxycarbonyl-propyl)-piperazino]-methyl-oxazolidin-2-on;
mit 3-[4-(1-Cyclohexyl-4-piperazinyl)-butyl]-5-methansulfonyloxy-methyl-oxazolidin-2-on das
3-[4-(1-Cyclohexyl-4-piperazinyl)-butyl]-5-[4-(3-methoxycarbonyl-propyl)-piperazino]-methyl-oxazolidin-2-on.

## Beispiel 4

0,6 g 3-(1-BOC-4-Piperidyl)-5-[4-(2-methoxycarbonyl-ethyl)-piperazino]-methyl-oxazolidin-2-on [erhältlich nach Bsp. 1] werden in 40 ml 2 N HCl-Lösung auf Dioxanbasis suspendiert und 3 Stunden bei Raumtemperatur gerührt. Nach Entfernung des Lösungsmittels und üblicher Aufarbeitung erhält man 3-(4-Piperidyl)-5-[4-(2-methoxycarbonyl-ethyl)-piperazino]-methyl-oxazolidin-2-on, Hydrochlorid.

Analog erhält man nach Entfernung der BOC-Schutzgruppe der Produkte aus Beispiel 1
3-(1-Amidino-4-piperidyl)-5-[4-(2-methoxycarbonyl-ethyl)-piperazino]-methyl-oxazolidin-2-on;
3-(1-Amidino-4-piperidylmethyl)-5-[4-(2-methoxycarbonyl-ethyl)-piperazino]-methyl-oxazolidin-2-on;
3-[2-(1-Amidino-4-piperidyl)-ethyl]-5-[4-(2-methoxycarbonyl-ethyl)-piperazino]-methyl-oxazolidin-2-on;
3-[3-(1-Amidino-4-piperidyl)-propyl]-5-[4-(2-methoxycarbonyl-ethyl)-piperazino]-methyl-oxazolidin-2-on;
3-[4-(1-Amidino-4-piperidyl)-butyl]-5-[4-(2-methoxycarbonyl-ethyl)-piperazino]-methyl-oxazolidin-2-on;
3-[2-(1-Amidino-4-piperazinyl)-ethyl]-5-[4-(2-methoxycarbonyl-ethyl)-piperazino]-methyl-oxazolidin-2-on;
3-[3-(1-Amidino-4-piperazinyl)-propyl]-5-[4-(2-methoxycarbonyl-ethyl)-piperazino]-methyl-oxazolidin-2-on;
3-[4-(1-Amidino-4-piperazinyl)-butyl]-5-[4-(2-methoxycarbonyl-ethyl)-piperazino]-methyl-oxazolidin-2-on.

## Beispiel 5

Analog Beispiel 4 erhält man ausgehend von den Verbindungen aus Beispiel 3 durch Abspaltung der BOC-Schutzgruppen die folgenden Verbindungen in Form ihrer Hydrochloride:
3-(4-Piperidyl)-5-[4-(methoxycarbonyl-methyl)-piperazino]-methyl-oxazolidin-2-on;
3-(1-Amidino-4-piperidyl)-5-[4-(methoxycarbonyl-methyl)-piperazino]-methyl-oxazolidin-2-on;
3-(1-Amidino-4-piperidylmethyl)-5-[4-(methoxycarbonyl-methyl)-piperazino]-methyl-oxazolidin-2-on;
3-[2-(1-Amidino-4-piperidyl)-ethyl]-5-[4-(methoxycarbonyl-methyl)-piperazino]-methyl-oxazolidin-2-on;
3-[3-(1-Amidino-4-piperidyl)-propyl]-5-[4-(methoxycarbonyl-methyl)-piperazino]-methyl-oxazolidin-2-on;
3-[4-(1-Amidino-4-piperidyl)-butyl]-5-[4-(methoxycarbonyl-methyl)-piperazino]-methyl-oxazolidin-2-on;
3-[2-(1-Amidino-4-piperazinyl)-ethyl]-5-[4-(methoxycarbonyl-methyl)-piperazino]-methyl-oxazolidin-2-on;
3-[3-(1-Amidino-4-piperazinyl)-propyl]-5-[4-(methoxycarbonyl-methyl)-piperazino]-methyl-oxazolidin-2-on;
3-[4-(1-Amidino-4-piperazinyl)-butyl]-5-[4-(methoxycarbonyl-methyl)-piperazino]-methyl-oxazolidin-2-on;
3-(1-Amidino-4-piperidyl)-5-[4-(3-methoxycarbonyl-propyl)-piperazino]-methyl-oxazolidin-2-on;
3-(1-Amidino-4-piperidylmethyl)-5-[4-(3-methoxycarbonyl-propyl)-piperazino]-methyl-oxazolidin-2-on;
3-[2-(1-Amidino-4-piperidyl)-ethyl]-5-[4-(3-methoxycarbonyl-propyl)-piperazino]-methyl-oxazolidin-2-on;
3-[3-(1-Amidino-4-piperidyl)-propyl]-5-[4-(3-methoxycarhonyl-propyl)-piperazino]-methyl-oxazolidin-2-on;
3-[4-(1-Amidino-4-piperidyl)-butyl]-5-[4-(3-methoxycarbonyl-propyl)-piperazino]-methyl-oxazolidin-2-on;
3-[2-(1-Amidino-4-piperazinyl)-ethyl]-5-[4-(3-methoxycarbonyl-propyl)-piperazino]-methyl-oxazolidin-2-on;
3-[3-(1-Amidino-4-piperazinyl)-propyl]-5-[4-(3-methoxycarbonyl-propyl)-piperazino]-methyl-oxazolidin-2-on;
3-[4-(1-Amidino-4-piperazinyl)-butyl]-5-[4-(3-methoxycarbonyl-propyl)-piperazino]-methyl-oxazolidin-2-on.

## Beispiel 6

0,8 g 3-(1-BOC-4-Piperidyl)-5-[4-(2-methoxycarbonyl-ethyl)-piperazino]-methyl-oxazolidin-2-on [erhältlich nach Bsp. 1] werden in 60 ml Methanol suspendiert, mit 4 ml 2 N NaOH-Lösung versetzt und 4 Stunden bei Raumtemperatur gerührt. Nach Entfernung des Lösungsmittels wird der Rückstand in Wasser aufgenommen, der pH-Wert durch Zugabe von verdünnter HCl auf 3 eingestellt und die Reaktionsmischung über einen Ionenaustauscher filtriert. Das Filtrat wird über $MgSO_4$ getrocknet. Nach Entfernung des Lösungsmittels und anschließende Gefriertrocknung erhält man 3-(1-BOC-4-Piperidyl)-5-[4-(2-carboxy-ethyl)-piperazino]-methyl-oxazolidin-2-on.

Analog erhält man durch Verseifung der Produkte aus den Beispielen 1 - 5:
3-(1-Benzyl-4-piperidyl)-5-[4-(2-carboxy-ethyl)-piperazino]-methyl-oxazolidin-2-on;
3-(1-N-BOC-Amidino-4-piperidyl)-5-[4-(2-carboxy-ethyl)-piperazino]-methyl-oxazolidin-2-on;
3-(1-Benzyl-4-piperidylmethyl)-5-[4-(2-carboxy-ethyl)-piperazino]-methyl-oxazolidin-2-on;
3-(1-N-BOC-Amidino-4-piperidylmethyl)-5-[4-(2-carboxy-ethyl)-piperazino]-methyl-oxazolidin-2-on;
3-[2-(1-Benzyl-4-piperidyl)-ethyl]-5-[4-(2-carboxy-ethyl)-piperazino]-methyl-oxazolidin-2-on;
3-[2-(1-N-BOC-Amidino-4-piperidyl)-ethyl]-5-[4-(2-carboxy-ethyl)-piperazino]-methyl-oxazolidin-2-on;
3-[3-(1-Benzyl-4-piperidyl)-propyl]-5-[4-(2-carboxy-ethyl)-piperazino]-methyl-oxazolidin-2-on;
3-[3-(1-N-BOC-Amidino-4-piperidyl)-propyl]-5-[4-(2-carboxy-ethyl)-piperazino]-methyl-oxazolidin-2-on;

3-[4-(1-Benzyl-4-piperidyl)-butyl]-5-[4-(2-carboxy-ethyl)-piperazino]-methyl-oxazolidin-2-on;
3-[4-(1-N-BOC-Amidino-4-piperidyl)-butyl]-5-[4-(2-carboxy-ethyl)-piperazino]-methyl-oxazolidin-2-on;
3-[2-(1-Benzyl-4-piperazinyl)-ethyl]-5-[4-(2-carboxy-ethyl)-piperazino]-methyl-oxazolidin-2-on;
3-[2-(1-N-BOC-Amidino-4-piperazinyl)-ethyl]-5-[4-(2-carboxy-ethyl)-piperazino]-methyl-oxazolidin-2-on;
3-[3-(1-Benzyl-4-piperazinyl)-propyl]-5-[4-(2-carboxy-ethyl)-piperazino]-methyl-oxazolidin-2-on;
3-[3-(1-N-BOC-Amidino-4-piperazinyl)-propyl]-5-[4-(2-carboxy-ethyl)-piperazino]-methyl-oxazolidin-2-on;
3-[4-(1-Benzyl-4-piperazinyl)-butyl]-5-[4-(2-carboxy-ethyl)-piperazino]-methyl-oxazolidin-2-on;
3-[4-(1-N-BOC-Amidino-4-piperazinyl)-butyl]-5-[4-(2-carboxy-ethyl)-piperazino]-methyl-oxazolidin-2-on;
3-(1-Methyl-4-piperidyl)-5-[4-(2-carboxy-ethyl)-piperazino]-methyl-oxazolidin-2-on;
3-(1-Isopropyl-4-piperidyl)-5-[4-(2-carboxy-ethyl)-piperazino]-methyl-oxazolidin-2-on;
3-(1-tert.-Butyl-4-piperidylmethyl)-5-[4-(2-carboxy-ethyl)-piperazino]-methyl-oxazolidin-2-on;
3-(1-Ethyl-4-piperidylmethyl)-5-[4-(2-carboxy-ethyl)-piperazino]-methyl-oxazolidin-2-on;
3-[2-(1-Isopropyl-4-piperidyl)-ethyl]-5-[4-(2-carboxy-ethyl)-piperazino]-methyl-oxazolidin-2-on;
3-[2-(1-Cyclohexyl-4-piperidyl)-ethyl]-5-[4-(2-carboxy-ethyl)-piperazino]-methyl-oxazolidin-2-on;
3-[3-(1-Methyl-4-piperidyl)-propyl]-5-[4-(2-carboxy-ethyl)-piperazino]-methyl-oxazolidin-2-on;
3-[3-(1-Cyclopentyl-4-piperidyl)-propyl]-5-[4-(2-carboxy-ethyl)-piperazino]-methyl-oxazolidin-2-on;
3-[4-(1-tert.-Butyl-4-piperidyl)-butyl]-5-[4-(2-carboxy-ethyl)-piperazino]-methyl-oxazolidin-2-on;
3-[4-(1-Cyciopropyl-4-piperidyl)-butyl]-5-[4-(2-carboxy-ethyl)-piperazino]-methyl-oxazolidin-2-on;
3-[2-(1-Methyl-4-piperazinyl)-ethyl]-5-[4-(2-carboxy-ethyl)-piperazino]-methyl-oxazolidin-2-on;
3-[2-(1-Cyclopropyl-4-piperazinyl)-ethyl]-5-[4-(2-carboxy-ethyl)-piperazino]-methyl-oxazolidin-2-on;
3-[3-(1-Ethyl-4-piperazinyl)-propyl]-5-[4-(2-carboxy-ethyl)-piperazino]-methyl-oxazolidin-2-on;
3-[3-(1-Isopropyl-4-piperazinyl)-propyl]-5-[4-(2-carboxy-ethyl)-piperazino]-methyl-oxazolidin-2-on;
3-[4-(1-Propyl-4-piperazinyl)-butyl]-5-[4-(2-carboxy-ethyl)-piperazino]-methyl-oxazolidin-2-on;
3-[4-(1-Cyclohexyl-4-piperazinyl)-butyl]-5-[4-(2-carboxy-ethyl)-piperazino]-methyl-oxazolidin-2-on.
3-(1-Benzyl-4-piperidyl)-5-[4-(2-carboxy-ethyl)-piperazino]-methyl-oxazolidin-2-on;
3-(1-Benzyl-4-piperidylmethyl)-5-[4-(2-carboxy-ethyl)-piperazino]-methyl-oxazolidin-2-on;
3-[2-(1-Benzyl-4-piperidyl)-ethyl]-5-[4-(2-carboxy-ethyl)-piperazino]-methyl-oxazolidin-2-on;
3-[3-(1-Benzyl-4-piperidyl)-propyl]-5-[4-(2-carboxy-ethyl)-piperazino]-methyl-oxazolidin-2-on;
3-[4-(1-Benzyl-4-piperidyl)-butyl]-5-[4-(2-carboxy-ethyl)-piperazino]-methyl-oxazolidin-2-on;
3-(1-BOC-4-Piperidyl)-5-[4-(carboxy-methyl)-piperazino]-methyl-oxazolidin-2-on;
3-(1-Benzyl-4-piperidyl)-5-[4-(carboxy-methyl)-piperazino]-methyl-oxazolidin-2-on;
3-(1-N-BOC-Amidino-4-piperidyl)-5-[4-(carboxy-methyl)-piperazino]-methyl-oxazolidin-2-on;
3-(1-Benzyl-4-piperidylmethyl)-5-[4-(carboxy-methyl)-piperazino]-methyl-oxazolidin-2-on;
3-(1-N-BOC-Amidino-4-piperidylmethyl)-5-[4-(carboxy-methyl)-piperazino]-methyl-oxazolidin-2-on;
3-[2-(1-Benzyl-4-piperidyl)-ethyl]-5-[4-(carboxy-methyl)-piperazino]-methyl-oxazolidin-2-on;
3-[2-(1-N-BOC-Amidino-4-piperidyl)-ethyl]-5-[4-(carboxy-methyl)-piperazino]-methyl-oxazolidin-2-on;
3-[3-(1-Benzyl-4-piperidyl)-propyl]-5-[4-(carboxy-methyl)-piperazino]-methyl-oxazolidin-2-on;
3-[3-(1-N-BOC-Amidino-4-piperidyl)-propyl]-5-[4-(carboxy-methyl)-piperazino]-methyl-oxazolidin-2-on;
3-[4-(1-Benzyl-4-piperidyl)-butyl]-5-[4-(carboxy-methyl)-piperazino]-methyl-oxazolidin-2-on;
3-[4-(1-N-BOC-Amidino-4-piperidyl)-butyl]-5-[4-(carboxy-methyl)-piperazino]-methyl-oxazolidin-2-on;
3-[2-(1-Benzyl-4-piperazinyl)-ethyl]-5-[4-(carboxy-methyl)-piperazino]-methyl-oxazolidin-2-on;
3-[2-(1-N-BOC-Amidino-4-piperazinyl)-ethyl]-5-[4-(carboxy-methyl)-piperazino]-methyl-oxazolidin-2-on;
3-[3-(1-Benzyl-4-piperazinyl)-propyl]-5-[4-(carboxy-methyl)-piperazino]-methyl-oxazolidin-2-on;
3-[3-(1-N-BOC-Amidino-4-piperazinyl)-propyl]-5-[4-(carboxy-methyl)-piperazino]-methyl-oxazolidin-2-on;
3-[4-(1-Benzyl-4-piperazinyl)-butyl]-5-[4-(carboxy-methyl)-piperazino]-methyl-oxazolidin-2-on;
3-[4-(1-N-BOC-Amidino-4-piperazinyl)-butyl]-5-[4-(carboxy-methyl)-piperazino]-methyl-oxazolidin-2-on;
3-(1-Methyl-4-piperidyl)-5-[4-(carboxy-methyl)-piperazino]-methyl-oxazolidin-2-on;
3-(1-Isopropyl-4-piperidyl)-5-[4-(carboxy-methyl)-piperazino]-methyl-oxazolidin-2-on;
3-(1-tert.-Butyl-4-piperidylmethyl)-5-[4-(carboxy-methyl)-piperazino]-methyl-oxazolidin-2-on;
3-(1-Ethyl-4-piperidylmethyl)-5-[4-(carboxy-methyl)-piperazino]-methyl-oxazolidin-2-on;
3-[2-(1-Isopropyl-4-piperidyl)-ethyl]-5-[4-(carboxy-methyl)-piperazino]-methyl-oxazolidin-2-on;
3-[2-(1-Cyclohexyl-4-piperidyl)-ethyl]-5-[4-(carboxy-methyl)-piperazino]-methyl-oxazolidin-2-on;
3-[3-(1-Methyl-4-piperidyl)-propyl]-5-[4-(carboxy-methyl)-piperazino]-methyl-oxazolidin-2-on;
3-[3-(1-Cyclopentyl-4-piperidyl)-propyl]-5-[4-(carboxy-methyl)-piperazino]-methyl-oxazolidin-2-on;
3-[4-(1-tert.-Butyl-4-piperidyl)-butyl]-5-[4-(carboxy-methyl)-piperazino]-methyl-oxazolidin-2-on;
3-[4-(1-Cyclopropyl-4-piperidyl)-butyl]-5-[4-(carboxy-methyl)-piperazino]-methyl-oxazolidin-2-on;
3-[2-(1-Methyl-4-piperazinyl)-ethyl]-5-[4-(carboxy-methyl)-piperazino]-methyl-oxazolidin-2-on;
3-[2-(1-Cyclopropyl-4-piperazinyl)-ethyl]-5-[4-(carboxy-methyl)-piperazino]-methyl-oxazolidin-2-on;
3-[3-(1-Ethyl-4-piperazinyl)-propyl]-5-[4-(carboxy-methyl)-piperazino]-methyl-oxazolidin-2-on;

3-[3-(1-Isopropyl-4-piperazinyl)-propyl]-5-[4-(carboxy-methyl)-piperazino]-methyl-oxazolidin-2-on;
3-[4-(1-Propyl-4-piperazinyl)-butyl]-5-[4-(carboxy-methyl)-piperazino]-methyl-oxazolidin-2-on;
3-[4-(1-Cyclohexyl-4-piperazinyl)-butyl]-5-[4-(carboxy-methyl)-piperazino]-methyl-oxazolidin-2-on;
3-(1-Benzyl-4-piperidyl)-5-[4-(3-carboxy-propyl)-piperazino]-methyl-oxazolidin-2-on;
3-(1-N-BOC-Amidino-4-piperidyl)-5-[4-(3-carboxy-propyl)-piperazino]-methyl-oxazolidin-2-on;
3-(1-Benzyl-4-piperidylmethyl)-5-[4-(3-carboxy-propyl)-piperazino]-methyl-oxazolidin-2-on;
3-(1-N-BOC-Amidino-4-piperidylmethyl)-5-[4-(3-carboxy-propyl)-piperazino]-methyl-oxazolidin-2-on;
3-[2-(1-Benzyl-4-piperidyl)-ethyl]-5-[4-(3-carboxy-propyl)-piperazino]-methyl-oxazolidin-2-on;
3-[2-(1-N-BOC-Amidino-4-piperidyl)-ethyl]-5-[4-(3-carboxy-propyl)-piperazino]-methyl-oxazolidin-2-on;
3-[3-(1-Benzyl-4-piperidyl)-propyl]-5-[4-(3-carboxy-propyl)-piperazino]-methyl-oxazolidin-2-on;
3-[3-(1-N-BOC-Amidino-4-piperidyl)-propyl]-5-[4-(3-carboxy-propyl)-piperazino]-methyl-oxazolidin-2-on;
3-[4-(1-Benzyl-4-piperidyl)-butyl]-5-[4-(3-carboxy-propyl)-piperazino]-methyl-oxazolidin-2-on;
3-[4-(1-N-BOC-Amidino-4-piperidyl)-butyl]-5-[4-(3-carboxy-propyl)-piperazino]-methyl-oxazolidin-2-on;
3-[2-(1-Benzyl-4-piperazinyl)-ethyl]-5-[4-(3-carboxy-propyl)-piperazino]-methyl-oxazolidin-2-on;
3-[2-(1-N-BOC-Amidino-4-piperazinyl)-ethyl]-5-[4-(3-carboxy-propyl)-piperazino]-methyl-oxazolidin-2-on;
3-[3-(1-Benzyl-4-piperazinyl)-propyl]-5-[4-(3-carboxy-propyl)-piperazino]-methyl-oxazolidin-2-on;
3-[3-(1-N-BOC-Amidino-4-piperazinyl)-propyl]-5-[4-(3-carboxy-propyl)-piperazino]-methyl-oxazolidin-2-on;
3-[4-(1-Benzyl-4-piperazinyl)-butyl]-5-[4-(3-carboxy-propyl)-piperazino]-methyl-oxazolidin-2-on;
3-[4-(1-N-BOC-Amidino-4-piperazinyl)-butyl]-5-[4-(3-carboxy-propyl)-piperazino]-methyl-oxazolidin-2-on;
3-(1-Methyl-4-piperidyl)-5-[4-(3-carboxy-propyl)-piperazino]-methyl-oxazolidin-2-on;
3-(1-Isopropyl-4-piperidyl)-5-[4-(3-carboxy-propyl)-piperazino]-methyl-oxazolidin-2-on;
3-(1-tert.-Butyl-4-piperidylmethyl)-5-[4-(3-carboxy-propyl)-piperazino]-methyl-oxazolidin-2-on;
3-(1-Ethyl-4-piperidylmethyl)-5-[4-(3-carboxy-propyl)-piperazino]-methyl-oxazolidin-2-on;
3-[2-(1-Isopropyl-4-piperidyl)-ethyl]-5-[4-(3-carboxy-propyl)-piperazino]-methyl-oxazolidin-2-on;
3-[2-(1-Cyclohexyl-4-piperidyl)-ethyl]-5-[4-(3-carboxy-propyl)-piperazino]-methyl-oxazolidin-2-on;
3-[3-(1-Methyl-4-piperidyl)-propyl]-5-[4-(3-carboxy-propyl)-piperazino]-methyl-oxazolidin-2-on;
3-[3-(1-Cyclopentyl-4-piperidyl)-propyl]-5-[4-(3-carboxy-propyl)-piperazino]-methyl-oxazolidin-2-on;
3-[4-(1-tert.-Butyl-4-piperidyl)-butyl]-5-[4-(3-carboxy-propyl)-piperazino]-methyl-oxazolidin-2-on;
3-[4-(1-Cyclopropyl-4-piperidyl)-butyl]-5-[4-(3-carboxy-propyl)-piperazino]-methyl-oxazolidin-2-on;
3-[2-(1-Methyl-4-piperazinyl)-ethyl]-5-[4-(3-carboxy-propyl)-piperazino]-methyl-oxazolidin-2-on;
3-[2-(1-Cyclopropyl-4-piperazinyl)-ethyl]-5-[4-(3-carboxy-propyl)-piperazino]-methyl-oxazolidin-2-on;
3-[3-(1-Ethyl-4-piperazinyl)-propyl]-5-[4-(3-carboxy-propyl)-piperazino]-methyl-oxazolidin-2-on;
3-[3-(1-Isopropyl-4-piperazinyl)-propyl]-5-[4-(3-carboxy-propyl)-piperazino]-methyl-oxazolidin-2-on;
3-[4-(1-Propyl-4-piperazinyl)-butyl]-5-[4-(3-carboxy-propyl)-piperazino]-methyl-oxazolidin-2-on;
3-[4-(1-Cyclohexyl-4-piperazinyl)-butyl]-5-[4-(3-carboxy-propyl)-piperazino]-methyl-oxazolidin-2-on;
3-(4-Piperidyl)-5-[4-(2-carboxy-ethyl)-piperazino]-methyl-oxazolidin-2-on, F.217-218°;
3-(1-Amidino-4-piperidyl)-5-[4-(2-carboxy-ethyl)-piperazino]-methyl-oxazolidin-2-on, F. > 300°;
3-(1-Amidino-4-piperidylmethyl)-5-[4-(2-carboxy-ethyl)-piperazino]-methyl-oxazolidin-2-on;
3-[2-(1-Amidino-4-piperidyl)-ethyl]-5-[4-(2-carbory-ethyl)-piperazino]-methyl-oxazolidin-2-on;
3-[3-(1-Amidino-4-piperidyl)-propyl]-5-[4-(2-carboxy-ethyl)-piperazino]-methyl-oxazolidin-2-on;
3-[4-(1-Amidino-4-piperidyl)-butyl]-5-[4-(2-carboxy-ethyl)-piperazino]-methyl-oxazolidin-2-on;
3-[2-(1-Amidino-4-piperazinyl)-ethyl]-5-[4-(2-carboxy-ethyl)-piperazino]-methyl-oxazolidin-2-on;
3-[3-(1-Amidino-4-piperazinyl)-propyl]-5-[4-(2-carboxy-ethyl)-piperazino]-methyl-oxazolidin-2-on;
3-[4-(1-Amidino-4-piperazinyl)-butyl]-5-[4-(2-carboxy-ethyl)-piperazino]-methyl-oxazolidin-2-on;
3-(4-Piperidyl)-5-[4-(carboxy-methyl)-piperazino]-methyl-oxazolidin-2-on;
3-(1-Amidino-4-piperidyl)-5-[4-(carboxy-methyl)-piperazino]-methyl-oxazolidin-2-on;
3-(1-Amidino-4-piperidylmethyl)-5-[4-(carboxy-methyl)-piperazino]-methyl-oxazolidin-2-on;
3-[2-(1-Amidino-4-piperidyl)-ethyl]-5-[4-(carboxy-methyl)-piperazino]-methyl-oxazolidin-2-on;
3-[3-(1-Amidino-4-piperidyl)-propyl]-5-[4-(carboxy-methyl)-piperazino]-methyl-oxazolidin-2-on;
3-[4-(1-Amidino-4-piperidyl)-butyl]-5-[4-(carboxy-methyl)-piperazino]-methyl-oxazolidin-2-on;
3-[2-(1-Amidino-4-piperazinyl)-ethyl]-5-[4-(carboxy-methyl)-piperazino]-methyl-oxazolidin-2-on;
3-[3-(1-Amidino-4-piperazinyl)-propyl]-5-[4-(carboxy-methyl)-piperazino]-methyl-oxazolidin-2-on;
3-[4-(1-Amidino-4-piperazinyl)-butyl]-5-[4-(carboxy-methyl)-piperazino]-methyl-oxazolidin-2-on;
3-(1-Amidino-4-piperidyl)-5-[4-(3-carboxy-propyl)-piperazino]-methyl-oxazolidin-2-on;
3-(1-Amidino-4-piperidylmethyl)-5-[4-(3-carboxy-propyl)-piperazino]-methyl-oxazolidin-2-on;
3-[2-(1-Amidino-4-piperidyl)-ethyl]-5-[4-(3-carboxy-propyl)-piperazino]-methyl-oxazolidin-2-on;
3-[3-(1-Amidino-4-piperidyl)-propyl]-5-[4-(3-carboxy-propyl)-piperazino]-methyl-oxazolidin-2-on;
3-[4-(1-Amidino-4-piperidyl)-butyl]-5-[4-(3-carboxy-propyl)-piperazino]-methyl-oxazolidin-2-on;
3-[2-(1-Amidino-4-piperazinyl)-ethyl]-5-[4-(3-carboxy-propyl)-piperazino]-methyl-oxazolidin-2-on;

3-[3-(1-Amidino-4-piperazinyl)-propyl]-5-[4-(3-carboxy-propyl)-piperazino]-methyl-oxazolidin-2-on;
3-[4-(1-Amidino-4-piperazinyl)-butyl]-5-[4-(3-carboxy-propyl)-piperazino]-methyl-oxazolidin-2-on.

**Beispiel 7**

Zu einer Lösung von 0,3 g 3-(1-Amidino-4-piperidyl)-5-[4-(2-carboxyethyl)-piperazino]-methyl-oxazolidin-2-on in 20 ml THF fügt man 10 ml 20%ige NaOH-Lösung hinzu und rührt 24 Std. bei Raumtemperatur. Man erhält nach Entfernung des Lösungsmittels und Gefriertrocknung 3-(1-Amidino-4-piperidyl)-5-[4-(2-carboxy-ethyl)-piperazino]-methyl-oxazolidin-2-on-Na-Salz.

Analog erhält man
3-[2-(1-Amidino-4-piperidyl)-ethyl]-5-[4-(2-carboxy-ethyl)-piperazino]-methyl-oxazolidin-2-on-Na-Salz;
3-[2-(1-Benzyl-4-piperidyl)-ethyl]-5-[4-(2-carboxy-ethyl)-piperazino]-methyl-oxazolidin-2-on-Na-Salz;
3-[2-(1-Amidino-4-piperazinyl)-ethyl]-5-[4-(2-carboxy-ethyl)-piperazino]-methyl-oxazolidin-2-on-Na-Salz;
3-[3-(4-Piperidyl)-propyl]-5-[4-(2-carboxy-ethyl)-piperazino]-methyl-oxazolidin-2-on-Na-Salz.

**Beispiel 8**

1,13 g 3-[2-(1-(5-Oxo-1,2,4-oxadiazolin-3-yl)-4-piperazinyl)-ethyl]-5-[4-(2-carboxy-ethyl)-piperazino]-methyl-oxazolidin-2-on [erhältlich nach Bsp. 1 durch Umsetzung von 3-Piperazino-propansäuremethyl-ester mit 3-[2-(1-(5-Oxo-1,2,4-oxadiazolin-3-yl)-4-piperazinyl)-ethyl]-5-methan-sulfonylmethyl-oxazolidin-2-on und anschließende Verseifung des Produktes] werden in 50 ml Methanol gelöst und an Raney-Nickel hydriert. Anschließend wird die Reaktionsmischung filtriert und das Filtrat im Vakuum eingeengt. Das erhaltene Produkt wird mit 20 ml Essigsäure-ethylester in der Wärme behandelt und nach Abkühlen abgesaugt. Man erhält 3-[2-(1-Amidino-4-piperazinyl)-ethyl]-5-[4-(2-carboxy-ethyl)-piperazino]-methyl-oxazolidin-2-on.

Analog erhält man durch reduktive Spaltung der 5-Oxo-1,2,4-oxadiazolin-Gruppe
aus 3-[3-(1-(5-Oxo-1,2,4-oxadiazolin-3-yl)-4-piperazinyl)-propyl]-5-[4-(2-carboxy-ethyl)-piperazino]-methyl-oxazolidin-2-on das
3-[3-(1-Amidino-4-piperazinyl)-propyl]-5-[4-(2-carboxy-ethyl)-piperazino]-methyl-oxazolidin-2-on;
aus 3-[4-(1-(5-Oxo-1,2,4-oxadiazolin-3-yl)-4-piperazinyl)-butyl]-5-[4-(2-carboxy-ethyl)-piperazino]-methyl-oxazolidin-2-on das
3-[4-(1-Amidino-4-piperazinyl)-butyl]-5-[4-(2-carboxy-ethyl)-piperazino]-methyl-oxazolidin-2-on;
aus 3-[2-(1-(5-Oxo-1,2,4-oxadiazolin-3-yl)-4-piperazinyl)-ethyl]-5-[4-(carboxy-methyl)-piperazino]-methyl-oxazolidin-2-on das
3-[2-(1-Amidino-4-piperazinyl)-ethyl]-5-[4-(carboxy-methyl)-piperazino]-methyl-oxazolidin-2-on;
aus 3-[3-(1-(5-Oxo-1,2,4-oxadiazolin-3-yl)-4-piperazinyl)-propyl]-5-[4-(carboxy-methyl)-piperazino]-methyl-oxazolidin-2-on das
3-[3-(1-Amidino-4-piperazinyl)-propyl]-5-[4-(carboxy-methyl)-piperazino]-methyl-oxazolidin-2-on;
aus 3-[4-(1-(5-Oxo-1,2,4-oxadiazolin-3-yl)-4-piperazinyl)-butyl]-5-[4-(carboxy-methyl)-piperazino]-methyl-oxazolidin-2-on das
3-[4-(1-Amidino-4-piperazinyl)-butyl]-5-[4-(carboxy-methyl)-piperazino]-methyl-oxazolidin-2-on;
aus 3-[2-(1-(5-Oxo-1,2,4-oxadiazolin-3-yl)-4-piperazinyl)-ethyl]-5-[4-(3-carboxy-propyl)-piperazino]-methyl-oxazolidin-2-on das
3-[2-(1-Amidino-4-piperazinyl)-ethyl]-5-[4-(3-carboxy-propyl)-piperazino]-methyl-oxazolidin-2-on;
aus 3-[3-(1-(5-Oxo-1,2,4-oxadiazolin-3-yl)-4-piperazinyl)-propyl]-5-[4-(3-carboxy-propyl)-piperazino]-methyl-oxazolidin-2-on das
3-[3-(1-Amidino-4-piperazinyl)-propyl]-5-[4-(3-carboxy-propyl)-piperazino]-methyl-oxazolidin-2-on;
aus 3-[4-(1-(5-Oxo-1,2,4-oxadiazolin-3-yl)-4-piperazinyl)-butyl]-5-[4-(3-carboxy-propyl)-piperazino]-methyl-oxazolidin-2-on das
3-[4-(1-Amidino-4-piperazinyl)-butyl]-5-[4-(3-carboxy-propyl)-piperazino]-methyl-oxazolidin-2-on.

**Beispiel 9**

Analog Beispiel 8 erhält man ausgehend von 3-[2-(1-(5-Oxo-1,2,4-oxadiazolin-3-yl)-4-piperidyl)-ethyl]-5-[4-(2-carboxy-ethyl)-piperazino]-methyl-oxazolidin-2-on [erhältlich nach Bsp. 1 durch Umsetzung von 3-(1-Piperazinyl)-propansäuremethyl-ester mit 3-[2-(1-(5-Oxo-1,2,4-oxadiazolin-3-yl)-4-piperidyl)-ethyl]-5-methansulfonylmethyl-oxazolidin-2-on und anschließende Verseifung des Produktes] durch katalytische Hydrierung das 3-[2-(1-Amidino-4-piperidyl)-ethyl]-5-[4-(2-carboxy-ethyl)-piperazino]-methyl-oxazolidin-2-on.

Analog erhält man durch reduktive Spaltung der 5-Oxo-1,2,4-oxadiazolin-Gruppe
aus 3-[3-(1-(5-Oxo-1,2,4-oxadiazolin-3-yl)-4-piperidyl)-propyl]-5-[4-(2-carboxy-ethyl)-piperazino]-methyl-oxazolidin-2-on das

3-[3-(1-Amidino-4-piperidyl)-propyl]-5-[4-(2-carboxy-ethyl)-piperazino]-methyl-oxazolidin-2-on;

aus 3-[4-(1-(5-Oxo-1,2,4-oxadiazolin-3-yl)-4-piperidyl)-butyl]-5-[4-(2-carboxy-ethyl)-piperazino]-methyl-oxazolidin-2-on das

3-[4-(1-Amidino-4-piperidyl)-butyl]-5-[4-(2-carboxy-ethyl)-piperazino]-methyl-oxazolidin-2-on;

aus 3-[2-(1-(5-Oxo-1,2,4-oxadiazolin-3-yl)-4-piperidyl)-ethyl]-5-[4-(carboxy-methyl)-piperazino]-methyl-oxazolidin-2-on das

3-[2-(1-Amidino-4-piperidyl)-ethyl]-5-[4-(carboxy-methyl)-piperazino]-methyl-oxazolidin-2-on;

aus 3-[3-(1-(5-Oxo-1,2,4-oxadiazolin-3-yl)-4-piperidyl)-propyl]-5-[4-(carboxy-methyl)-piperazino]-methyl-oxazolidin-2-on das

3-[3-(1-Amidino-4-piperidyl)-propyl]-5-[4-(carboxy-methyl)-piperazino]-methyl-oxazolidin-2-on;

aus 3-[4-(1-(5-Oxo-1,2,4-oxadiazolin-3-yl)-4-piperidyl)-butyl]-5-[4-(carboxy-methyl)-piperazino]-methyl-oxazolidin-2-on das

3-[4-(1-Amidino-4-piperidyl)-butyl]-5-[4-(carboxy-methyl)-piperazino]-methyl-oxazolidin-2-on;

aus 3-[2-(1-(5-Oxo-1,2,4-oxadiazolin-3-yl)-4-piperidyl)-ethyl]-5-[4-(3-carboxy-propyl)-piperazino]-methyl-oxazolidin-2-on das

3-[2-(1-Amidino-4-piperidyl)-ethyl]-5-[4-(3-carboxy-propyl)-piperazino]-methyl-oxazolidin-2-on;

aus 3-[3-(1-(5-Oxo-1,2,4-oxadiazolin-3-yl)-4-piperidyl)-propyl]-5-[4-(3-carboxy-propyl)-piperazino]-methyl-oxazolidin-2-on das

3-[3-(1-Amidino-4-piperidyl)-propyl]-5-[4-(3-carboxy-propyl)-piperazino]-methyl-oxazolidin-2-on;

aus 3-[4-(1-(5-Oxo-1,2,4-oxadiazolin-3-yl)-4-piperidyl)-butyl]-5-[4-(3-carboxy-propyl)-piperazino]-methyl-oxazolidin-2-on das

3-[4-(1-Amidino-4-piperidyl)-butyl]-5-[4-(3-carboxy-propyl)-piperazino]-methyl-oxazolidin-2-on.

Die nachfolgenden Beispiele betreffen pharmazeutische Zubereitungen:

**Beispiel A: Injektionsgläser**

Eine Lösung von 100 g eines Wirkstoffes der Formel I und 5 g Dinatriumhydrogenphosphat werden in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2 H_2O$, 28,48 g $Na_2HPO_4 \cdot 12 H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I

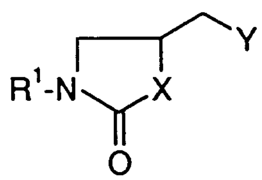

I

worin

X O, S, NH oder NA,

Y einen durch $R^2$ substituierten Aziridino-, Azetidino-, Pyrrolidino-, Piperidino-, Hexahydroazepino- oder Piperazinorest,

$R^1$

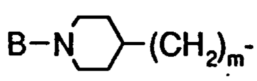 oder 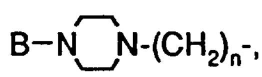

$R^2$ $-C_rH_{2r}-COOR^3$,

$R^3$ H, A oder Ar,

A Alkyl mit 1 bis 6 C-Atomen,

B H, A, Cycloalkyl mit 3 bis 7 C-Atomen, $Ar-C_kH_{2k}-$ oder Amidino,

Ar einen unsubstituierten oder ein- oder zweifach durch A, Cl, Br, I, $NO_2$, CN, OA, OH, $NH_2$, NHA und/oder $NA_2$ substituierten Phenyl- oder Benzyl-Rest,

k 1, 2, 3 oder 4,

m und r jeweils unabhängig voneinander 0, 1, 2, 3 oder 4

und

n 2, 3 oder 4

bedeuten,
sowie ihre physiologisch unbedenklichen Salze.

2. Enantiomere oder Diastereomere der Verbindungen der Formel I gemäß Anspruch 1.

3. Verbindungen der Formel I gemäß Anspruch 1, worin die freien Amino- oder Amidino-gruppen teilweise oder vollständig durch konventionelle Amino-Schutzgruppen geschützt sind.

4.

(a) 3-Piperidino-4-yl-5-[4-(2-carboxyethyl)-piperazinomethyl]-oxazolidin-2-on;

(b) 3-(1-Amidinopiperidin-4-yl)-5-[4-(2-carboxyethyl)-piperazinomethyl]-oxazolidin-2-on;

(c) 3-(1-Benzylpiperidin-4-yl)-5-[4-(2-methoxycarbonylethyl)-piperazinomethyl]-oxazolidin-2-on;

(d) 3-(1-Benzylpiperidin-4-yl)-5-(2-benzyloxycarbonylethyl)-piperazinomethyl]-oxazolidin-2-on;

(e) 3-(2-Piperidin-4-yl-ethyl)-5-[4-(3-carboxypropyl)-piperazinomethyl]-oxazolidin-2-on;

(f) 3-(1-Amidinopiperidin-4-yl-methyl)-5-(4-carboxymethyl)-piperazinomethyl]-oxazolidin-2-on;

(g) 3-[3-(1-Amidinopiperazin-4-yl)-propyl]-5-(4-carboxymethyl-piperazinomethyl)-oxazolidin-2-on,

sowie deren physiologisch unbedenklichen Salze.

5. Verfahren zur Herstellung von Verbindungen der Formel I gemäß Anspruch 1, dadurch gekennzeichnet, daß man

(a) eine Verbindung der Formel I aus einem ihrer funktionellen Derivate durch Behandeln mit einem solvolysierenden oder hydrogenolysierenden Mittel in Freiheit setzt, oder daß man

(b) eine Verbindung der Formel II

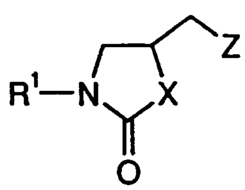

II,

worin

$R^1$ und X die in Anspruch 1 angegebenen Bedeutungen besitzen

und

Z Cl, Br, I, OH oder eine reaktionsfähig veresterte OH-Gruppe bedeutet,

mit einer Verbindung der Formel III

H-Y III,

worin

Y die angegebene Bedeutung besitzt,

umsetzt, oder daß man

(c) eine Verbindung der Formel IV

$R^1$-NH-$CH_2$-CH(XH)-$CH_2$-Y IV,

worin

$R^1$, X und Y die angegebenen Bedeutungen haben,

mit einen reaktionsfähigen Derivat der Kohlensäure umsetzt, oder daß man

(d) eine Verbindung der Formel V

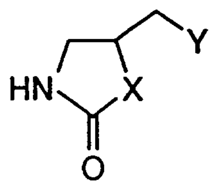

V,

worin

X und Y die angegebenen Bedeutungen haben, mit einer Verbindung der Formel VI

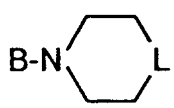

VI,

worin

B die angegebene Bedeutung hat

und

L

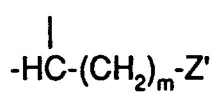

oder N-$(CH_2)_n$-Z',

wobei

Z' Cl, Br, I, OA, OH oder eine reaktionsfähige veresterte OH-Gruppe bzw. leicht nucleophil substituierbare Abgangsgruppe bedeutet,

umsetzt, oder daß man

(e) (einen) Rest(e) $R^1$ und/oder Y in (einen) andere(n) Rest(e) $R^1$ und/oder Y umwandelt, und/oder daß man

(f) eine Verbindung der Formel I durch Behandeln mit einer Säure oder Base in eines ihrer Salze überführt.

6. Verfahren zur Herstellung einer pharmazeutischen Zubereitung, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

7. Pharmazeutische Zubereitung, gekennzeichnet durch einen Gehalt an mindestens einer Verbindung der Formel I, gemäß Anspruch 1, und/oder einem ihrer physiologisch unbedenklichen Salze.

8. Verwendung einer Verbindung der Formel I gemäß Anspruch 1 und/oder eines ihrer physiologisch unbedenklichen Salze zur Herstellung von Arzneimitteln.

9. Verwendung einer Verbindung der Formel I gemäß Anspruch 1 und/oder eines ihrer physiologisch unbedenklichen Salze bei der Bekämpfung von Krankheiten.

Europäisches Patentamt

# EUROPÄISCHER RECHERCHENBERICHT

Nummer der Anmeldung
EP 95 11 6889

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | DE-A-44 05 633 (MERCK PATENT GMBH)<br>* das ganze Dokument *<br>--- | 1,7 | C07D413/06<br>C07D417/06<br>A61K31/40 |
| A,D | EP-A-0 459 256 (MERCK PATENT GMBH)<br>* Seite 2 *<br>--- | 1,7 | |
| A | EP-A-0 443 197 (MERCK PATENT GMBH)<br>* Seite 2 *<br>--- | 1,7 | |
| A | EP-A-0 300 272 (MERCK PATENT GMBH)<br>* Seite 2 - Seite 4, Zeile 6 *<br>----- | 1,7 | |

RECHERCHIERTE SACHGEBIETE (Int.Cl.6)

C07D

Der vorliegende Recherchenbericht wurde für alle Patentansprüche erstellt

| Recherchenort | Abschlußdatum der Recherche | Prüfer |
|---|---|---|
| BERLIN | 16.Februar 1996 | Kyriakakou, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)